# EUROPÄISCHE PATENTSCHRIFT

(11) **EP 1 392 808 B1**
(45) Veröffentlichungstag und Bekanntmachung des Hinweises auf die Patenterteilung: **30.08.2006**
(21) Anmeldenummer: 02740629.7
(22) Anmeldetag: 22.05.2002
(51) Int. Cl.: C11D 1/06, C11D 1/08, C11D 1/83, A61K 8/39, A61Q 19/00

(54) **ALPHA-HYDROXYSÄUREESTERHALTIGE TENSIDZUBEREITUNGEN**
SURFACTANT PREPARATIONS COMPRISING ALPHA-HYDROXY ACID ESTERS
COMPOSITIONS TENSIOACTIVES CONTENANT DES ESTERS D'ACIDES ALPHA-HYDROXY

(30) Priorität: 30.05.2001 DE 10126196
(43) Veröffentlichungstag der Anmeldung: 03.03.2004
(73) Patentinhaber: Cognis IP Management GmbH, 40589 Düsseldorf (DE)
(72) Erfinder: BEHLER, Ansgar, 46240 Bottrop (DE); SEIPEL, Werner, 40723 Hilden (DE); KUBLIK, Heike, 47906 Kempen (DE)
(86) Internationale Anmeldenummer: PCT/EP2002/005587
(87) Internationale Veröffentlichungsnummer: WO 2002/097022

(56) Entgegenhaltungen:
- WO-A-01/60332
- WO-A-97/25965
- CA-A- 1 087 955
- DE-A- 19 622 214
- US-A- 3 662 054
- US-A- 3 962 150
- US-B1- 6 190 675

## Beschreibung

### Gebiet der Erfindung

Die Erfindung betrifft Tensidzubereitungen, enthaltend alpha-Hydroxysäureester mit ethoxylierten Alkoholen, sich durch eine verbesserte Haut- und Schleimhautverträglichkeit auszeichnen, sowie die Verwendung von Hydroxycarbonsäureestern mit ethoxylierten Alkoholen zur Reduzierung des Irritationspotentials von Tensiden.

### Stand der Technik

Bei der Entwicklung neuer Tenside steht der praktische Verbraucherschutz an erster Stelle. Immer wieder erfährt man, dass Tenside mit sehr guten anwendungstechnischen Eigenschaften nicht die Kriterien der lokalen Verträglichkeit erfüllen. Insbesondere von anionischen Tensiden ist bekannt, dass sie ein hohes Irritationspotential aufweisen [Kästner, W. und Frosch, J.P., Hautirritationen verschiedener anionaktiver Tenside im Duhring-Kammer-Test am Menschen im Vergleich zu in vitro- und tierexperimentellen Methoden in Fette, Seifen, Anstrichmittel 83, 1981 Seite 33 - 46]. Dabei soll die lokale Verträglichkeit abhängig sein von der C-Kettenlänge und dem Ethoxylierungsgrad. Insbesondere Kettenlängen von 10 bis 14 Kohlenstoffatomen und Tenside mit geringem Ethoxylierungsgrad zeigen eine weniger gute Hautverträglichkeit. Man kann diese Kenntnisse in die Produktentwicklung einfließen lassen, muß allerdings dann häufig einen Verlust guter anwendungstechnischer Eigenschaften in Kauf nehmen.

Es besteht also auch heutzutage trotz einer Vielzahl gut verträglicher Tenside auf dem Markt immer noch Bedarf an Substanzen, die diese Eigenschaften miteinander kombinieren und trotz guter waschaktiver Eigenschaften, Kompatibilität mit anderen Hilfsstoffen und optimaler Verarbeitbarkeit auch eine sehr gute Verträglichkeit aufweisen.

Die Aufgabe der vorliegenden Erfindung hat daher darin bestanden, das Irritationspotential von Tensiden zu senken und Zubereitungen mit anionischen Tensiden zur Verfügung zu stellen, die eine hohe Oberflächenaktivität haben und trotzdem eine verbesserte Haut-, Schleimhaut- und Gewebeverträglichkeit aufweisen.

### Beschreibung der Erfindung

Gegenstand der Erfindung ist die Verwendung von Estern von Hydroxycarbonsäuren mit ethoxylierten Alkoholen zur Verbesserung der Haut-, Schleimhaut- und Gewebeverträglichkeit von anionischen Tensiden, sowie das Verfahren zur Verbesserung der Haut-, Schleimhaut- und Gewebeverträglichkeit von anionischen Tensiden, dadurch gekennzeichnet, dass man den Tensiden oder Tensidlösungen Estern von Hydroxycarbonsäuren mit ethoxylierten Alkoholen zusetzt.

Ein weiterer Gegenstand der Erfindung sind Zubereitungen, enthaltend
(a) anionische Tenside und
(b) 25 bis 50 Gew.% Ester von alpha-Hydroxycarbonsäuren mit ethoxylierten Alkoholen der Formel (III) bezogen auf die Menge an anionischen Tensiden,
dadurch gekennzeichnet, dass sie eine Oberflächenaktivität von mindestens 20 mN/m aufweisen (gemessen bei Raumtemperatur nach der Wilhelmy-Platten-Methode (Platin-Platte PT11 nach DIN 53914) und dass sie im HET-CAM-Test einen Q-Wert von weniger als 75% bezogen auf die alpha-Hydroxycarbonsäurefreie Tensidlösung aufweisen.

Überraschenderweise wurde gefunden, dass der Zusatz von Hydroxycarbonsäureestern mit ethoxylierten Alkoholen zu anionischen Tensiden das Irritationspotential der Tenside stark vermindert und damit die Haut-, Schleimhaut- und Gewebeverträglichkeit erheblich verbessert ohne dass ein Verlust an Waschaktivität festzustellen ist.
Zubereitungen mit anionischen Tensiden in Kombination mit alpha-Hydroxycarbonsäureestern mit ethoxylierten Alkoholen zeigen eine wesentlich bessere Verträglichkeit als die reinen Tensidformulierungen, dadurch gekennzeichnet, dass der Reaktionszeitwert im HET-CAM-Test um mindestens 25% verbessert werden kann, bezogen auf den Ausgangswert der reinen Tensidformulierung.
Diese Tatsache war auch deshalb nicht zu erwarten, weil gerade in antimikrobiellen Reinigungsmitteln antimikrobielle Wirkstoffe und anionische Tenside mit Carbonsäuren kombiniert werden, um eine bessere antimikrobielle Wirksamkeit zu erreichen. Dabei soll - wie in der internationalen Patentanmeldung **WO 98/55093** auf Seite 10 beschrieben - die Säure die negative Ladung der Bakterienzellwände reduzieren und die Zellmembran, die durch das Tensid geschwächt ist, leicht überwinden, um den pH-Wert im Cytoplasma der Zelle zu verschieben. Ein Mechanismus, der übertragen auf die Zellen der menschlichen Haut sicher nicht auf eine gute Verträglichkeit schließen läßt.

### HET-CAM-Test

Der HET-CAM-Test, Hühner-Ei-Test-Chorioallantoismembran-Test, stellt einen Test auf akute schleimhautreizende Wirkung von Substanzen auf das Gefäßsystem der Chorioallantoismembran (CAM) von befruchteten und 10 Tage lang bebrüteten Hühnereiern dar [Köszegi, Dunja et al. : Der HET-CAM-Test, Euro Cosmetics 11/12-99, S. 29 - 33, Künstler, Klaus: Alternative Methoden in der Industrie am Beispiel der Haut- und Schleimhautverträglichkeitsprüfung in Schuppan/Hardegg, Tierschutz durch Alternativen, Gustav Fischer Verlag, Stuttgart 1988].
Die sich während der Bebrütung entwickelnde Chorioallantoismembran enthält ein funktionstüchtiges Gefäßsystem. Sie wird durch Entfernen der Eischale oberhalb der Luftkammer freigelegt und mit 300 µl der zu testenden Lösung überdeckt. Die Reaktionen des Gefäßsystems auf die Substanzen werden bis zu einem Zeitraum von 300 Sekunden beobachtet. Die Bewertung der Reizstärke erfolgt nach Art und zeitlichem Eintreten. Die Reaktionszeit ist die Zeit in Sekunden zwischen Applikation und Auftreten des Reaktionsparameters. Als Reaktionsparameter werden Hämorrhagie (Blutung), Lyse (Auflösung der Blutgefäße) und Koagulation (Eiweißgerinnung) beurteilt. Über die Reaktionszeit wird ein Reizwert [Q] als Maßzahl für die jeweilige Prüfsubstanz im Vergleich zu einer Referenzsubstanz (Texapon ASV, 5 % Aktivsubstanz) erstellt.

| **Reizwert [Q]** | **Reizstärke** |
|---|---|
| 0,8 | Schwach reizend |
| ≤ 0,8 < 1,2 | Mäßig reizend |
| ≥ 1,2 < 2,0 | Reizend |
| ≥ 2,0 | Stark reizend |

Die Zugabe von Estern von Hydroxycarbonsäuren mit ethoxylierten Alkoholen soll bei der vorliegenden Erfindung das Irritationspotential von anionischen Tensiden merklich reduzieren. Der Q-Wert der hydroxycarbonsäureester freien Tensidzubereitung soll nach Zugabe der Ester mit ethoxylierten Alkoholen nur noch 75% des Ausgangswertes betragen, bevorzugt ist sogar eine Senkung auf 70 % und besonders bevorzugt auf 65 % des Wertes der hydroxycarbonsäureester freien Formulierung.

### Bestimmung der Oberflächenaktivität

Die Bestimmung der Oberflächenaktivität erfolgte nach nach DIN 53914. Dazu wurde die zu untersuchende Lösung bei 21±1 °C mit der Wilhelmy-Platten-Methode geprüft (Platte PT11)
Die erfindungsgemäßen Zubereitungen sollen eine Oberflächenaktivität von mindestens 20 mN/m, bevorzugt mindestens 25 mN/m und besonders bevorzugt mindestens 30 mN/m aufweisen bezogen auf den Aktivgehalt von 5 Gew. %.

### Anionische Tenside

Typische Beispiele für anionische Tenside der erfindungsgemäßen Zubereitungen sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.
Bevorzugt werden in den erfindungsgemäßen Zubereitungen jedoch Alkylsulfate, Fettalkoholethersulfate, Alkansulfonate und Alkylsulfosuccinaten, besonders bevorzugt darunter die Alkyl- und/oder Alkenylsulfate, sowie die Alkylethersulfate.

Unter Alkyl- und/oder Alkenylsulfaten, die auch häufig als Fettalkoholsulfate bezeichnet werden, sind die Sulfatierungsprodukte primärer Alkohole zu verstehen, die der Formel (I) folgen,

**R**^{**2**}**O-SO**_{**3**}**X** **(I)**

in der R² für einen linearen oder verzweigten, aliphatischen Alkyl- und/oder Alkenylrest mit 6 bis 22, vorzugsweise 12 bis 18 Kohlenstoffatomen und X für ein Alkali- und/oder Erdalkalimetall, Ammonium, Alkylammonium, Alkanolammonium oder Glucammonium steht. Typische Beispiele für Alkylsulfate, die im Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Die Sulfatierungsprodukte können vorzugsweise in Form ihrer Alkalisalze und insbesondere ihrer Natriumsalze eingesetzt werden. Besonders bevorzugt sind Alkylsulfate auf Basis von C_{16/18}-Talgfettalkoholen bzw. pflanzliche Fettalkohole vergleichbarer C-Kettenverteilung in Form ihrer Natriumsalze.

Alkylethersulfate ("Ethersulfate") stellen bekannte anionische Tenside dar, die großtechnisch durch SO₃- oder Chlorsulfonsäure (CSA)-Sulfatierung von Fettalkohol- oder Oxoalkoholpolyglycolethern und nachfolgende Neutralisation hergestellt werden. Im Sinne der Erfindung kommen Ethersulfate in Betracht, die der Formel (II) folgen,

**R**^{**1**}**O-(CH**_{**2**}**CH**_{**2**}**O)**_{**m**}**SO**_{**3**}**X** **(II)**

in der R¹ für einen linearen oder verzweigten Alkyl- und/oder Alkenylrest mit 6 bis 22 Kohlenstoffatomen, m für Zahlen von 0 oder 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 0 oder 1 bis 10 und insbesondere 1 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 1,5 bis 2,5 Mol Ethylenoxid an technische C_{12/14}- bzw. C_{17/18}- Kokosfettalkoholfraktionen in Form ihrer Natrium- und/oder Magnesiumsalze. In den erfindunggemäßen Zubereitungen liegen die anionischen Tenside in Mengen von 1 bis 99 Gew.%, vorzugsweise 2 bis 30 Gew. % und besonders bevorzugt von 10 bis 20 Gew.% bezogen auf die Menge an Aktivsubstanz vor.
Gegenstand der vorliegenden Erfindung sind somit auch Zubereitungen enthaltend
(a) 2 bis 30 Gew.% anionische Tenside und
(b) 25 bis 50 Gew.% Ester von alpha-Hydroxycarbonsäuren mit ethoxylierten Alkoholen der Formel (III) bezogen auf die Menge an anionischen Tensiden,
dadurch gekennzeichnet, dass sie eine Oberflächenaktivität von mindestens 20 mN/m aufweisen (gemessen bei Raumtemperatur nach der Wilhelmy-Platten-Methode (Platin-Platte PT11 nach DIN 53914) und dass sie im HET-CAM-Test einen Q-Wert von weniger als 75% bezogen auf die alpha-Hydroxycarbonsäurefreie Tensidlösung aufweisen.

### Hydroxycarbonsäuren und deren Salze und Ester mit ethoxylierten Alkoholen

Hydroxycarbonsäuren sind organische Säuren, die neben mindestens einer COOH-Gruppe im Molekül mindestens eine OH-Gruppe enthalten. Sie können als Monohydroxycarbonsäuren mit einer OH-Gruppe vorliegen, mit zwei als Di-, oder mit mehr als zwei OH-Gruppen als Polyhydroxycarbonsäuren vorliegen. Nach der Stellung der OH-Gruppe zur COOH-Gruppe unterscheidet man alpha-, beta-, und gamma-Hydroxycarbonsäuren.

Die Säuren können gesättigt oder ungesättigt (Beisp.: Ricinolsäure) vorkommen. Bekannte aromatische Hydroxycarbonsäuren sind die Salicylsäure (2-Hydroxybenzoesäure) und die Gallussäure (3,4,5-Trihydroxybenzoesäure).
In der vorliegenden Erfindung bevorzugte Hydroxycarbonsäuren, stellen alpha-Hydroxycarbonsäuren, darunter insbesondere die Weinsäure, Mandelsäure, Milchsäure, Äpfelsäure, Citronensäure und deren Salze.

Die Ester der Hydroxycarbonsäuren mit ethoxylierten Alkoholen stellen bekannte Verbindungen dar, die der Formel (III) folgen,

**R**^{**3**}**-(EO)**_{**x**}**-Z** **(III)**

in der R³ für einen Alkylrest mit 6 bis 22 C-Atomen, bevorzugt mit 12 bis 14 C-Atomen, X für einen ganzzahligen Wert von 2 bis 10 und Z für einen Hydroxycarbonsäurerest steht. In der vorliegenden Erfindung bevorzugte Hydroxycarbonsäuren als Rest Z, stellen alpha-Hydroxycarbonsäuren, darunter insbesondere die Weinsäure, Mandelsäure, Milchsäure, Äpfelsäure, Citronensäure dar.

Die erfindungsgemäßen Zubereitungen enthalten 25 bis 50 Gew.% Ester der Hydroxycarbonsäuren mit Alkylethoxylaten bezogen auf die Menge an anionischen Tensiden.

### Gewerbliche Anwendbarkeit

Die erfindungsgemäßen Zubereitungen sollen für die Herstellung von kosmetischen Mitteln, Zahnpflegemitteln, Wasch-, Spül- und Reinigungsmitteln, jedoch nicht ausschließlich in diesen Bereichen, eingesetzt werden.
Alle Produkte, durch die ein Kontakt der Haut mit anionischen Tensiden gegeben ist, können die erfindungsgemäßen Zubereitungen, enthaltend anionische Tenside in Kombination mit Estern der alpha-Hydroxycarbonsäuren mit ethoxylierten Alkoholen beinhalten, um ein geringeres Irritationsrisiko aufzuweisen.

Je nach Verwendungszweck enthalten die erfindungsgemäßen Zubereitungen weitere Hilfsstoffe, wobei möglichst auf die Zugabe von antimikrobiellen Wirkstoffen verzichtet werden soll. Sie können in fester Form, bevorzugt jedoch in wäßriger oder alkoholisch/wäßriger Lösung vorliegen. Der pH-Wert der Zubereitungen soll im Bereich zwischen pH 4 und pH 7, vorzugsweise zwischen pH 5 und pH 6,8, besonders bevorzugt zwischen pH 6 und pH 6,6 liegen.

Für kosmetische und/oder pharmazeutische Anwendungen können sie als weitere Hilfs- und Zusatzstoffe ferner andere milde Tenside, Emulgatoren, Rückfetter, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

Werden die erfindungsgemäßen Tensidgemische zur Herstellung von Wasch- und Reinigungsmitteln verwendet, so können die Zubereitungen noch weitere typische Inhaltsstoffe, wie beispielsweise Lösungsmittel, Hydrotrope, Bleichmittel, Bleichaktivatoren, Waschkraftverstärker, Builder, Viskositätsregulatoren, Enzyme, Enzymstabilisatoren, optische Aufheller, Soil repellants, Schauminhibitoren, anorganische Salze sowie Duft- und Farbstoffe aufweisen.

### Beispiele

### Bestimmung der Oberflächenspannung

Die Oberflächenspannung der weinsäurehaltigen Tensidlösung wurde mit der der reinen Tensidlösung (Texapon® NSO, Natriumlaurylethersulfat; Cognis, Düsseldorf) verglichen, indem Messungen bei 21±1°C mit der Wilhelmy-Platten-Methode (Platte PT11) nach der Methode DIN 53914 durchgeführt wurden.

**Tabelle 1a: Oberfächenspannung der weinsäurehaltigen und -freien Tensidlösung***

| **Substanz** | **Konzentration** | **Oberflächenspannung** |
|---|---|---|
| Texapon® NSO | 5 Gew.% Aktivsubstanz Natriumlaurylethersulfat | 31,6 mN/m |
| Texapon® NSO mit Weinsäure | 5 Gew.% Aktivsubstanz Natriumlaurylethersulfat 0,5 Gew.% Weinsäure | 30,3 mN/m |

| | | |
|---|---|---|
| * Die Beispiele fallen nicht unter den Schutzumfang des Ansprüche | | |

### Bestimmung des Schaumverhaltens

Zur Bestimmung des Schaumverhaltens wurde mit der Rotor-Schaum-Methode (DIN 13996 in Vorbereitung) die Anschäumkinetik nach 30 Sekunden und das Schaumpotential nach 60 s und 180 s gemessen.
Das Rotor-Schaumtestgerät besteht aus einem temperierbaren, doppelwandigen zylindrischen Glasgefäß mit einem Innendurchmesser von 17,5 cm. Auf dem zylindrischen Glasgefäß ist ein Maßstab mit mm-Einteilung zum Ablesen der Schaumhöhe und des Flüsigkeitsstandes angebracht. Femer ist das Glasgefäß mit einem Styropor-Deckel versehen, der zur Abdeckung und Isolierung des Gefäßes dient. Das Rührgerät besteht aus einem Spezialrührkopf mit 28 cm langer Rührwelle von 1 cm Durchmesser und einem *JK*-Rührwerk mit digitaler Drehzahlanzeige. Zusätzlich werden ein Thermostat, eine StoppUhr und ein Thermometer (digital) benötigt.

Zur Herstellung der Prüflösung wurde Wasser von definierter Härte (15°dH) eingesetzt.
200 ml der auf 30±1 °C vortemperierten Probe wurden langsam am Rand des Glasgefäßes eingegossen und nach Erreichen der Solltemperatur von 30±1 °C mit dem Styropordeckel abgedeckt. Die Drehzahl des Rotors betrug 1300 UpM.
Der erste Wert der Schaumhöhe wurde nach 30 Sekunden ermittelt, dazu wurde das Rührwerk für maximal 10 Sekunden abgestellt. Danach wurde die Schaummenge nach 60 und 180 Sekunden bestimmt.

**Tabelle 1b: Schaumverhalten der weinsäurehaltigen und -freien Tensidlösung**

| **Substanz** | **Konzentration** | **Schaumverhalten** | |
|---|---|---|---|
| Texapon® NSO | 0,1 Gew.% Aktivsubstanz | 30 s | 10,8 cm |
| | Natriumlaurylethersulfat | 60 s | 11,0 cm |
| | | 180s | 11,0 cm |
| Texapon® NSO mit Weinsäure | 0,1 Gew.% Aktivsubstanz | 30 s | 10,8 cm |
| | Natriumlaurylethersulfat | 60 s | 11,0 cm |
| | 0,01 Gew.% Weinsäure | 180 s | 11,0 cm |
| Texapon® NSO mit Laureth-4-citrat* | 0,1 Gew.% Aktivsubstanz | 30 s | 9,5 cm |
| | Natriumlaurylethersulfat | 60 s | 10,8 cm |
| | 0,01 Gew.% Laureth-4-citrat | 180 s | 11,0 cm |

| | | | |
|---|---|---|---|
| * erfindungsgemäßes Beispiel | | | |

Aus den Versuchsergebnissen (Tabellen 1a und 1b) geht deutlich hervor, dass die Zugabe von Weinsäure oder Laureth-4-citrat die Oberflächenaktivität und das Schaumverhalten des Aniontensides nicht merklich beeinflußt, so dass davon ausgegangen werden kann, dass die Zugabe von Weinsäure oder Laureth-4-citrat die Waschaktivität des Tensides nicht herabsetzt, obgleich das Irritationspotential vermindert wird.

### Bestimmung des Irritationspotentials - In-vitro-Prüfung: HET-CAM-Test, Reaktionszeitmethode

Zur Bestimmung des Irritationspotentials wurde ein HET-CAM-Test, wie beschrieben in "Der HET-CAM-Test", Euro Cosmetics 11/12-99, S. 29 - 33, Köszegi, Dunja et al. durchgeführt

**Tabelle 2: Reaktionszeitmethode und Endpunktbeurteilung nach dem HET-CAM-Test**

| **Substanz** | **Konzentration** | **Reaktionszeitwert [Q]** |
|---|---|---|
| Texapon® ASV | 5 Gew.% Aktivsubstanz Gemisch spezieller milder Fettalkoholethersulfate | 1,00 |
| Texapon® NSO | 5 Gew.% Aktivsubstanz Natriumlaurylethersulfat | 1,63 |
| Texapon® NSO | 3 Gew.% Aktivsubstanz Natriumlaurylethersulfat | 1,47 |
| Texapon® NSO mit Weinsäure | 5 Gew.% Aktivsubstanz Natriumlaurylethersulfat 0,5 Gew.% Weinsäure | 1,01 |
| Texapon® NSO mit Laureth-4-citrat* | 3,75 Gew.% Aktivsubstanz Natriumlaurylethersulfat 1,25 Gew.% Laureth-4-citrat | 0,95 |

| | | |
|---|---|---|
| erfindungsgemäßes Beispiel | | |

Die Ergebnisse (Tabelle 2) zeigen eine deutliche Reduktion des Irritationspotentials anionischer Tenside durch die Zugabe von Weinsäure oder Laureth-4-citrat. Der Reaktionszeitwert [Q] wird durch die Zugabe von 10 Gew.% Weinsäure (bezogen auf die Menge an anionischen Tensiden) auf 62 % des ursprünglichen Wertes gesenkt; durch die Zugabe von 33% Laureth-4-citrat (bezogen auf die Menge an anionischen Tensiden) sogar auf 58 % des ursprünglichen Wertes.

## Patentansprüche

1. Verwendung von Hydroxycarbonsäureestem mit ethoxylierten Alkoholen gemäß Formel (III),
R³O-(EO)ₓ-Z (III)
in der R³ für einen Alkylrest mit 6 bis 22 C-Atomen, X für einen ganzzahligen Wert von 2 bis 10 und Z für einen Hydroxycarbonsäurerest steht, zur Verbesserung der Haut-, Schleimhaut- und Gewebeverträglichkeit von anionischen Tensiden.

2. Verfahren zur Verbesserung der Haut-, Schleimhaut- und Gewebeverträglichkeit von anionischen Tensiden, **dadurch gekennzeichnet, dass** man den Tensiden oder Tensidlösungen Hydroxycarbonsäureester mit ethoxylierten Alkoholen zusetzt, die der Formel (III) folgen,
R³O-(EO)ₓ-Z (III)
in der R³ für einen Alkylrest mit 6 bis 22 C-Atomen, X für einen ganzzahligen Wert von 2 bis 10 und Z für einen Hydroxycarbonsäurerest steht.

3. Zubereitungen enthaltend
(a) anionische Tenside und
(b) 25 bis 50 Gew.% Ester von alpha-Hydroxycarbonsäuren mit ethoxylierten Alkoholen der Formel (III), bezogen auf die Menge an anionischen Tensiden,
R³O-(EO)ₓ-Z (III)
in der R³ für einen Alkylrest mit 6 bis 22 C-Atomen, X für einen ganzzahligen Wert von 2 bis 10 und Z für einen Hydroxycarbonsäurerest steht,
**dadurch gekennzeichnet, dass** sie eine Oberflächenaktivität von mindestens 20 mN/m aufweisen (gemessen bei 21±1 °C nach der Methode DIN 53914) und dass sie im HET-CAM-Test einen Q-Wert von weniger als 75% bezogen auf die alpha-Hydroxycarbonsäurefreie Tensidlösung aufweisen.

## Claims

1. The use of hydroxycarboxylic acid esters with ethoxylated alcohols corresponding to formula (III):
R³O-(EO)ₓ-Z (III)
in which R³ is an alkyl group containing 6 to 22 carbon atoms, X is an integer of 2 to 10 and Z is a hydroxycarboxylic acid residue,
for improving the compatibility of anionic surfactants with the skin, mucous membrane and tissue.

2. A process for improving the compatibility of anionic surfactants with the skin, mucous membrane and tissue, **characterized in that** hydroxycarboxylic acid esters with ethoxylated alcohols corresponding to formula (III):
R³O-(EO)ₓ- Z (III)
in which R³ is an alkyl group containing 6 to 22 carbon atoms, X is an integer of 2 to 10 and Z is a hydroxycarboxylic acid residue,
are added to the surfactants or surfactant solutions.

3. Preparations containing
(a) anionic surfactants and
(b) 25 to 50% by weight, based on the quantity of anionic surfactants, of esters of α-hydroxycarboxylic acids with ethoxylated alcohols corresponding to formula (III):
R³O-(EO)ₓ-Z (III)
in which R³ is an alkyl group containing 6 to 22 carbon atoms, X is an integer of 2 to 10 and Z is a hydroxycarboxylic acid residue,
**characterized in that** they have a surface activity of at least 20 mN/m (as measured at 21±1°C by the DIN 53914 method) and **in that** they have a Q value in the HET-CAM test of less than 75%, based on the surfactant solution without α-hydroxycarboxylic acid.

## Revendications

1. Utilisation d'esters d'acides hydroxycarboxyliques avec des alcools éthoxylés selon la formule (III),
R³O-(EO)ₓ-Z (III)
dans laquelle R³ représente un radical alkyle comportant 6 à 22 atomes de C, X représente un nombre entier de 2 à 10, et Z représente un radical acide hydroxycarboxylique, pour améliorer la compatibilité de tensioactifs anioniques avec la peau, les muqueuses et les tissus.

2. Procédé d'amélioration de la compatibilité de tensioactifs anioniques avec la peau, les muqueuses et les tissus,
**caractérisé en ce qu'**
on ajoute aux tensioactifs ou aux solutions tensioactives des esters d'acides hydroxycarboxyliques avec des alcools éthoxylés, de formule (III)
R³O-(EO)ₓ-Z (III)
dans laquelle R³ représente un radical alkyle comportant 6 à 22 atomes de C, X représente un nombre entier de 2 à 10 et Z représente un radical acide hydroxycarboxylique.

3. Préparations contenant :
(a) des tensioactifs anioniques et
(b) 25 à 50 % en poids d'esters d'acides alpha-hydroxycarboxyliques avec des alcools éthoxylés de formule (III), par rapport à la quantité de tensioactifs anioniques,
R³O-(EO)ₓ-Z (III)
dans laquelle R³ représente un radical alkyle comportant 6 à 22 atomes de C, X représente un nombre entier de 2 à 10, et Z représente un radical acide hydroxycarboxylique,
**caractérisées en ce qu'**
elles présentent une activité superficielle d'au moins 20 mN/m (mesurée à 21 ± 1 °C d'après la méthode DIN 53914), et au test HET-CAM, une valeur Q inférieure à 75 % par rapport à la solution tensioactive dépourvue d'acide alpha-hydroxycarboxylique.
